# EUROPEAN PATENT SPECIFICATION

(11) **EP 4 638 023 B1**
(45) Date of publication and mention of the grant of the patent: **18.11.2026**
(21) Application number: 23828379.0
(22) Date of filing: 12.12.2023
(51) Int. Cl.: B06B 1/02, G01H 11/06, G01N 29/26, A61B 8/00, A61B 8/08

(54) **RING ARRAY TRANSDUCER**
RINGARRAY-WANDLER
TRANSDUCTEUR À RÉSEAU ANNULAIRE

(30) Priority: 19.12.2022 EP 22214485
(43) Date of publication of application: 29.10.2025
(73) Proprietor: mems.works holding B.V., 4835 NJ Breda (NL)
(72) Inventor: DIRKSEN, Peter, 5656 AG Eindhoven (NL); SHULEPOV, Sergey Yuryevich, 5656 AG Eindhoven (NL); TIMMERMANS, Petrus Henricus Maria, 5656 AG Eindhoven (NL); VAN SCHAIJK, Rob, 5656 AG Eindhoven (NL); DE WIJS, Willem-Jan Arend, 5656 AG Eindhoven (NL)
(74) Representative: Arnold & Siedsma
(86) International application number: PCT/EP2023/085286
(87) International publication number: WO 2024/132674

(56) References cited:
- CN-A- 111 250 376
- US-A1- 2013 128 702
- US-B2- 7 257 051
- GULDIKEN R ET AL: "P0-18 Forward-Looking IVUS Imaging Using a Dual-Annular Ring CMUT Array: Experimental Results", ULTRASONICS SYMPOSIUM, 2007. IEEE, IEEE, PISCATAWAY, NJ, USA, 28 October 2007 (2007-10-28), pages 1247 - 1250, XP031999166, ISBN: 978-1-4244-1383-6, DOI: 10.1109/ULTSYM.2007.313
- CHIENLIU CHANG ET AL: "Singulation for imaging ring arrays of capacitive micromachined ultrasonic transducers", JOURNAL OF MICROMECHANICS AND MICROENGINEERING, INSTITUTE OF PHYSICS PUBLISHING, BRISTOL, GB, vol. 24, no. 10, 22 September 2014 (2014-09-22), pages 107002, XP020271306, ISSN: 0960-1317, [retrieved on 20140922], DOI: 10.1088/0960-1317/24/10/107002

## Description

### FIELD OF THE INVENTION

The invention relates to the field of ultrasound transducers. In particular, the invention relates to the field of ring array transducers.

### BACKGROUND OF THE INVENTION

Ring array transducers enable volumetric imaging while using a relatively low number of channels compared to conventional two-dimensional, 2D, array transducers. This means that fewer cables have to be connected to the transducer. More generally, a ring array is thought to be the simplest transducer geometry that combines three-dimensional, 3D, imaging with a low number of channels. Thus, it proves possible to get a 3D image using, for example, only 64 channels as the computational load is reduced with the lower number of elements.

For example, in Choe et. al. "GPU-based real-time volumetric ultrasound image reconstruction for a ring array", IEEE Transactions on Medical Imaging, 18 Mar 2013, 32(7):1258-1264, DOI: 10.1109/tmi.2013.2253117, a ring array is shown with 64 elements capable of 3D imaging. The ring array shown has a radius of 1.16mm with an imaging depth of 2mm - 15mm.

However, such an imaging depth is typically not enough for external imaging/monitoring applications. Further, it has been realized that the focal region of such a ring is limited and may not meet the requirements for external applications, such as fetal monitoring.

US 7,257,051 B2 discloses an integrated switch matrix for reconfiguring subelements of a mosaic sensor array to form elements. The configuration of the switch matrix is fully programmable and includes access switches that connect subelements to bus lines and matrix switches that connect subelements to subelements. Each subelement has a unit switch cell comprising at least one access switch, at least one matrix switch, a respective memory element for storing the future state of each switch, and a respective control circuit for each switch. The access and matrix switches are of a type having the ability to memorize control data representing the current switch state of the switch, which control data includes a data bit input to turn-on/off circuits incorporated in the control circuit.

### SUMMARY OF THE INVENTION

The invention is defined by the claims.

According to the invention, there is provided a transducer ring array for volumetric ultrasound imaging according to claim 1.

Ring arrays enable 3D ultrasound imaging with a low number of channels. However, the focal region of ring is limited and often does not meet the requirements for certain applications (e.g., fetal monitoring).

As such, it is proposed to generate multi-ring arrays while still having a low number of channels. For double-ring arrays, this can be achieved by having two different types of channels in the array. The first channels have at least one outer CMUT drum and at least one inner CMUT drum. The second channels have only an outer CMUT drum. That is, each second channels comprises at least one outer CMUT drum, but no inner CMUT drum.

The outer CMUT drums of both the first and second channels form an outer ring (e.g., similar to a single-ring array) while the inner CMUT drums of the first channel form a second, inner, ring. A biasing system is used to activate either the outer CMUT drums (for faraway imaging) or the inner CMUT drums (for nearby imaging). As such, the focal region of the ring array is increased without the need to add additional channels.

The first channels can be used to transmit/receive ultrasound signals from either the outer CMUT drums or the inner CMUT drums.

The CMUT drums may be placed on a substrate (e.g., a printed circuit board, PCB). The substrate may be a planar rigid substrate or a flexible substrate. When placed on a flexible substrate, the ring array may be arranged/placed on a curved structure.

The first channels may comprise two or more outer CMUT drums and the outer CMUT drums further from the at least one inner CMUT drum may have a larger diameter than the outer CMUT drums closer to the at least one inner CMUT drum.

Having the diameter of CMUT drums increase towards the outer edges of the ring array improves the packing density of the ring array. This is possible because the center frequency of a CMUT can be approximated as being independent of the diameter provided the other parameters (e.g., bias voltage) are constant.

The first channels may comprise two or more inner CMUT drums and the inner CMUT drums closer to the outer CMUT drums may have a larger diameter than the inner CMUT drums further from the outer CMUT drums.

The first channels may comprise three or more outer CMUT drums and three or more inner CMUT drums and the second channels may comprise three of more outer CMUT drums.

Having three or more CMUT drums per channel in both the inner and outer rings increases the transmit and receive signal strength, thereby leading to an increase in the quality of the ultrasound data obtained.

The biasing system may comprise two individually addressable connections for each first channel, one for the at least one outer CMUT drum and one for the at least one inner CMUT drum.

The biasing system may comprises a bias switching system on each first channel for switching between applying a biasing voltage to the at least one outer CMUT drum and the at least one inner CMUT drum of the first channels.

The invention also provides an ultrasound imaging system comprising:
the transducer ring array; and
a controller configured to:
   obtain nearby ultrasound data from the at least one inner CMUT drum (e.g., the one or more inner CMUT drums) of the first channels by applying a bias to the at least one inner CMUT drum;
   obtain faraway ultrasound data from the at least one outer CMUT drum (e.g., the one or more outer CMUT drums) of the first channels and the at least one outer CMUT drum (e.g., the one or more outer CMUT drums) of the second channels by applying a bias to the at least one outer CMUT drum of the first channels; and
   generate a compound, three dimensional, ultrasound image by combining the nearby ultrasound data and the faraway ultrasound data.

Obtaining faraway ultrasound data may also comprise applying a bias to the outer CMUT drums of the second channels.

The invention also provides a method of manufacturing a transducer ring array according to claim 6.

It has been realized that manufacturing ring shapes results in poor yields and high usage of silicon. As such, it is proposed to manufacture portions of the ring array and then combine the portions. This means that less silicon is used overall, and any deficient portions can be discarded individually, instead of having to discard the whole ring array.

Each second channel comprises only a plurality of outer CMUT drums.

However, the each second chanel does not comprise any inner CMUT drum.

The method may further comprise placing the ring array portions on a substrate in a ring pattern, the substrate comprising an RF line for each first channel, for connecting the outer CMUT drum and the inner CMUT drum of the corresponding first channel, and a biasing system for each of the first channels for applying a biasing voltage to either the outer CMUT drum or the inner CMUT drum of each first channel.

The substrate may also comprise an RF line for each second channel, connected to the outer CMUT drum of corresponding second channel, and a biasing system for each of the second channels for applying a biasing voltage to the outer CMUT drum of the corresponding second channel.

The method may further comprise combining four or more ring array portions to generate the transducer ring array.

The method may further comprise separating the ring array portions from the silicon wafer by using deep reactive ion etching, DRIE, to etch the silicon wafers and grinding the etched silicon wafers to release the ring array portions.

This is particularly advantageous when the ring array portions are non-rectangular (e.g., when the ring array portions are curved).

Of course, if the ring array portions are, for example, rectangular, the ring array portions can be obtained from the silicon wafer via dicing.

The method may further comprise testing the ring array channels to identify acceptable ring array portions and combining two or more of the acceptable ring portions to generate the transducer ring array.

Combining the two or more ring array portions comprises placing the ring array portions in a ring pattern.

Combining the two or more ring array portions may comprise placing the ring array portions on a substrate, the substrate comprising an RF line connecting the at least one outer CMUT drum and the at least one inner CMUT drum of each first channel and a biasing system for each of the first channels for applying a biasing voltage to either the at least one outer CMUT drum or the at least one inner CMUT drum of each first channel.

The substrate may be a flexible or non-flexible (e.g., flat) printed circuit board (PCB), depending on the end application requirements. For example, the substrate may be flexible such that it can be wrapped around a curved shape.

The biasing system may comprise two individually addressable connections for each first channel, one for the at least one outer CMUT drum and one for the at least one inner CMUT drum.

The biasing system may comprise a bias switching system on each first channel for switching between applying a biasing voltage to the at least one outer CMUT drum and at least one the inner CMUT drum of the first channels.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a sketch of a single ring array;
Fig. 2 shows contours of the focal regions for single ring arrays with different diameters;
Fig. 3 illustrates the concept of a multi ring array;
Fig. 4 shows a double ring array with 64 elements;
Fig. 5 shows part of a ring array with a dense pack ring array geometry;
Fig. 6 shows a silicon wafer with ring elements; and
Fig. 7 shows an assembled ring array with four ring array portions.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a transducer ring array for volumetric ultrasound imaging. The ring array comprises a set of first channels, each first channel comprising an outer CMUT drum and an inner CMUT drum connected via a radiofrequency, RF, line. A biasing system, for each first channel, is used to apply a biasing voltage to either the outer CMUT drum or the inner CMUT drum of the first channels. The ring array also comprises a set of second channels, each second channel comprises an outer CMUT drum. The outer CMUT drums of both the first channels and the second channels form an outer ring with an outer diameter, and the inner CMUT drums of the first channels form an inner ring with a smaller, inner, diameter.

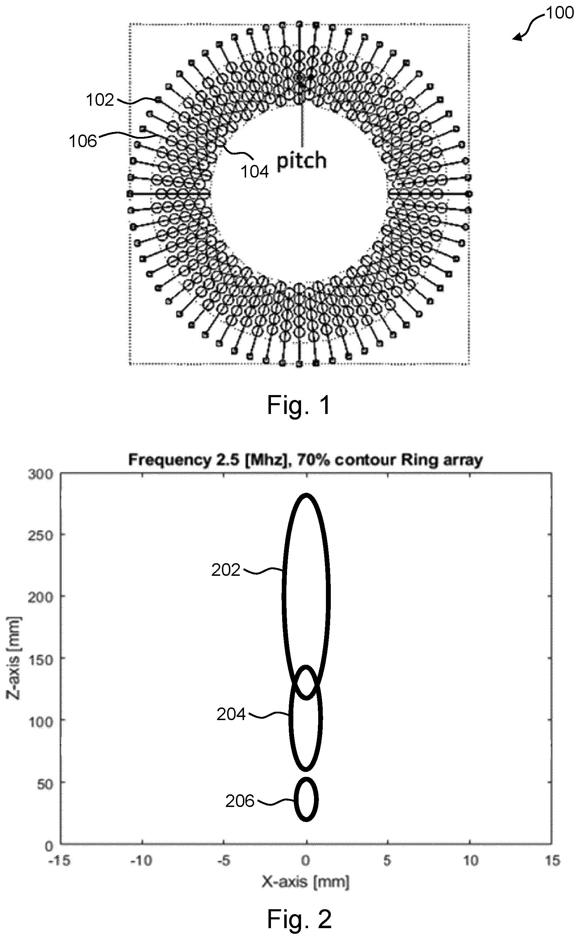

Fig. 1 shows a sketch of a single ring array 100. The single ring array 100 has 64 channels 102, where each channel 102 has five CMUT drums 10 connected with a radio frequency, RF, line 106. As such, only the 64 channels have to be analyzed.

There is an interest in monitoring solutions for pregnancies. In particular, there is an interest in monitoring solutions for high-risk pregnancies with preexisting complications.

However, the single ring array 100 has been found to have a limited focal region, thereby making it unsuitable for fetal imaging/monitoring. As a rule of thumb, the average pitch between the CMUT drums 104 limits the maximal center frequency for a ring array. It has been found that for a single ring array with a focal region up to around 280mm, a ring diameter of around 12mm is needed. Keeping the number of channels 102 to 64 means that the maximum center frequency is limited to around 2.2. MHz (assuming a two-pulse excitation and 40% bandwidth - acceptable for monitoring purposes).

The pitch generally refers to the period of a periodic structure. In this case, the distance between adjacent CMUT drums is called the pitch. For a ring array, the pitch (distance) is not a constant: outer CMUT elements are at a larger distance compared to the inner. However, assuming a 'thin' ring array, one may use the average pitch.

The relation between pitch, operating frequency and image quality is well known, both from an experimental point of view and theoretically. For a phased array, the general rule is that the pitch should be smaller than the wavelength *λ* in order to avoid grating lobes that spoil the image quality. Typically the pitch is chosen as smaller than half the wavelength *λ*. However, usually a somewhat larger pitch is still allowed. For example a pitch of 0.7*λ* is also used.

In this case, the wavelength corresponds to an upper-corner wavelength *λᵤₚ*. One often excites a transducer in a pulsed mode, the frequency spectrum having a certain width result. The central frequency is called *F_{c}*, the upper-corner frequency is called *Fᵤₚ* and the lower corner frequecny is called *F_{low}.* The bandwidth corresponds to the frequency difference between *Fᵤₚ* and *F_{low}.* A high bandwidth means that the transducer responds to a large range in frequency, which provide a good and crisp image.

Frequency and wavelength are related: *F* = *λc*, where *c* is the speed of sound. As such, a high frequency means a small wavelength, which also means a smaller pitch is needed. For water, the speed of sound is approximately 1500 meter/sec. Thus, the higher the desired upper corner frequency is, the smaller the pitch is required to be.

However, because of the limited focal region, such a single ring array would be limited to a minimum depth of around 120mm. For fetal monitoring, a penetration depth between ~280mm and ~80mm is required.

Fig. 2 shows contours 202, 204 and 206 of the focal regions for single ring arrays with different diameters. The x-axis shows a lateral distance relative to the single ring array (in mm). The z-axis shows a depth relative to the single ring array (in mm).

The contours are line of equal amplitude (70% of the maximum) of the absolute value of the pressure. The 70% contour indicated is generally of interest as this part is most suitable for imaging. Inside this contour, the pressure is at maximum.

The contour 202 shows the focal region for a single ring array with a diameter of 28mm at a frequency of 2.5Mhz. The contour 204 shows the focal region for a single ring array with a diameter of 20mm at a frequency of 2.5Mhz. The contour 206 shows the focal region for a single ring array with a diameter of 12mm at a frequency of 2.5Mhz.

As can be seen, none of the contours shown provided a large enough focal region for fetal monitoring/imaging (i.e., covering ~80mm to ~280mm).

The focal regions for single ring arrays with diameters of 26mm, 24mm, 16mm and 14mm were also determined (not shown). None of the single ring arrays proved sufficient for fetal monitoring (i.e., having a focal region covering ~80mm to ~280mm).

As such, there is a need for an improved solution which provides a higher focal region (e.g., for fetal monitoring) whilst keeping the number of channels to a minimum.

It is proposed to provide a multi-ring array where the CMUT drums of the inner ring(s) are connected to the same RF line as the part of the CMUT drums of the outer ring such than some channels comprise CMUT drums of the inner ring(s) and CMUT drums of the outer ring. A biasing system is then provided to either bias the CMUT drums of the outer ring or the CMUT drums of the inner ring. The outer ring and the inner ring(s) will have different diameters such that the multi-ring array essentially has two (or more) focal regions. As such, the combination of the focal regions provides an increased overall focal region.

This results in an effective focal region comprised of a combination of the contours shown in Fig. 2. For example, the contour 202 (having a diameter of 28mm) and the contour 204 (having a diameter of 20mm), when combined, could cover a focal region between ~80mm to ~280mm.

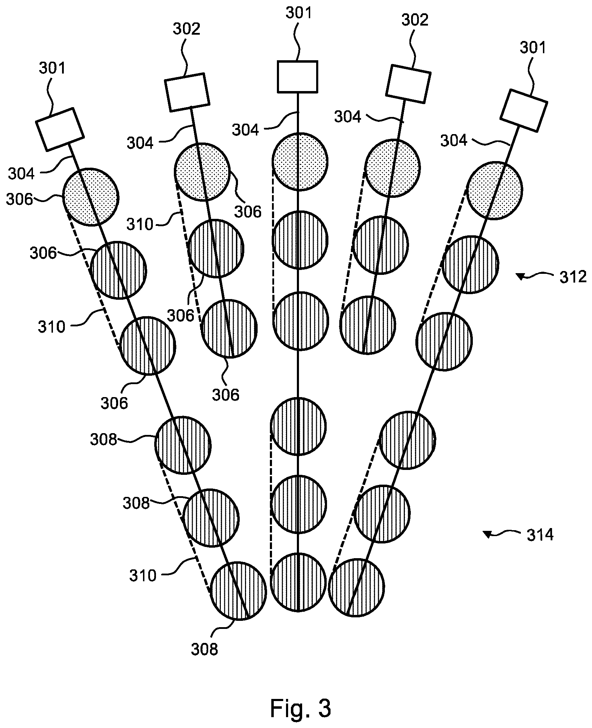

Fig. 3 illustrates the concept of a multi ring array. In particular, Fig. 3 shows part of a double ring array. Five channels are shown, three first channels 301 and two second channels 302. In this example, the first channels 301 have an RF line 304 connected to six CMUT drums; three outer CMUT drums 306 and three inner CMUT drums 308. The outer CMUT drums 306 are all connected to a biasing line 310 for applying a bias to the outer CMUT drums 306. The inner CMUT drums 308 are also connected to a biasing line 310 for biasing the inner CMUT drums 308. As such, a bias can be applied to either the outer CMUT drums 306 or the inner CMUT drums 308.

The second channels 302 have three outer CMUT drums 306, all connected to a biasing line 310 for applying a bias to the outer CMUT drums 306. The outer ring 312 is formed of the outer CMUT drums 306 of both the first channels 301 and the second channels 302 and the inner ring 314 is formed of the inner CMUT drums 308 of the first channels 301.

The outer ring 312, having an outer diameter, will have a first focal region, and the inner ring 314, having a smaller inner diameter, will have a second focal region. As such, ultrasound data obtained from both of the rings can be combined to provide ultrasound data for a larger, combined, focal region.

Any of the CMUT drums can be used in the collapsed or non-collapsed mode.

Bias switching between the rings can be used to select the appropriate ring. Next the data may can be combined into a single compound image. Alternatively, an independent bias line can be used for the outer CMUT drums 306 and the inner CMUT drums 308. It is noted that bias switching may need fewer connections than having independent bias lines.

A biasing system using bias switching involves a circuit capable of only providing a bias to either the outer CMUT drums 306 or the inner CMUT drums 308 of the first channels 301.

A double or multiple ring array can thus provide nearby imaging (with the inner ring 314) and far away imaging (with the outer ring 312). For nearby imaging, the bias is turned on for the inner CMUT drums 308 and turned off for the outer CMUT drums 306. In other words, during nearby imaging, it is as if only the CMUT drums of the inner ring 314 are active and the CMUT drums of the outer ring 312 are not present at all.

For far away imaging, the bias is turned off for the inner CMUT drums 308 and is only turned on for the outer CMUT drums 306. Compound ultrasound data can thus be obtained by combining the ultrasound data obtained from the nearby imaging and the far away imaging. For example, a compound 3D image can be generated by combining the images from nearby imaging the far away imaging. The compound ultrasound data/image will thus be able to over the focal regions of both the outer ring 312 and the inner ring 314.

Thus, a double or multiple ring array solves the focal region issue. Additionally, the diameters of the outer ring 312 and the inner ring 314 can be selected to cover, for example, an entire penetration depth of 50mm to 250mm without increasing the complexity of the electronics (i.e., the number of channels remains low).

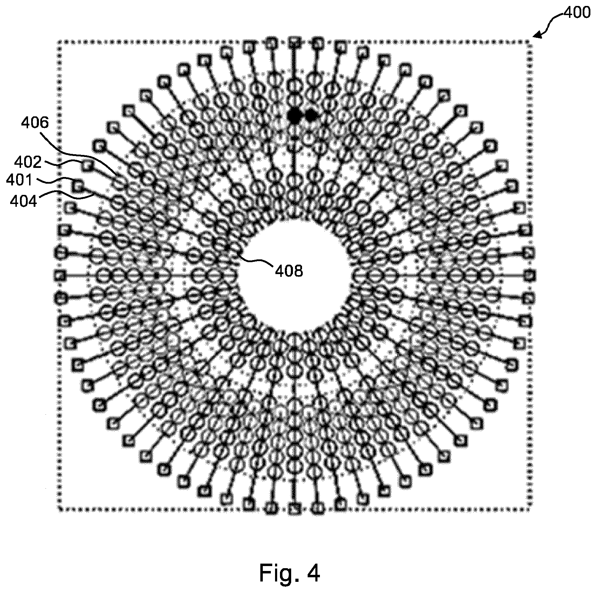

Fig. 4 shows a double ring array 400 with 64 elements. The double ring array 400 has 32 first channels 401 and 32 second channels 402. The first channels 401 each have five outer CMUT drums 406 and three inner CMUT drums 408 connected via an RF line. The second channels 402 each have five outer CMUT drums 406 connected via an RF line 404. A biasing system (not shown) is used which can bias either the outer CMUT drums 406 or the inner CMUT drums 408 of the first channels 401. As such, the double ring array 400 can be switched between using the outer ring (i.e., the outer CMUT drums 406 of both the first channels 401 and the second channels 402) or the inner ring (i.e., the inner CMUT drums 308 of the first channels 401).

The double ring array 400 includes the integration of scanning CMUT MEMS ultrasound transducer. Because a single US transducer plane is not able to cover the whole womb, multiple US transducers (e.g., distributed over the abdomen for fetal monitoring) are used and are integrated in a conformal patch and/or smart textile. The double ring array can thus be used to identify fetus movement, position, anatomy and, detects mother and fetus heart rate due to its larger overall focal range.

As previously discussed, a single ring array will not cover the required focal depth for fetal monitoring. This issue is solved by using a double ring, where either the inner ring is activated by switching on the bias voltage or the outer ring is activated. Thus, a larger depth of focus is obtained. Note that the number of channels is not increased and is fully determined by the outer ring.

Note that, for Fig. 4, an open circle represents a single CMUT drum (e.g., having a typical diameter of about 350 microns in the case of low frequency CMUT drums ~ 2.5MHz). The squares on each channel represent bond pads. Not all components are shown (e.g., the bias lines).

A multi-ring array can be used in both professional and non-professional settings (e.g., for a remote monitoring at home).

Note that Fig. 4 shows a flat double ring array. However, the ring array may be placed on a flexible substrate such that it can be wrapped around a curved structure.

A 'dense pack ring array' geometry is further proposed to improve the performance.

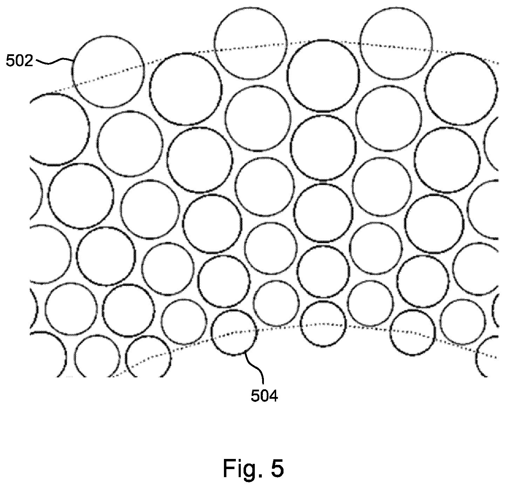

Fig. 5 shows part of a ring array with a dense pack ring array geometry. The packing density of a ring array may be improved by having a CMUT diameter that increases towards the rim of the array. In other words, the CMUT drums 502 further from the center of the ring(s) have a larger diameter than the CMUT drums 504 closer to the center of the ring.

To a fair approximation, the center frequency of a CMUT is independent of the diameter, provided all other parameters including the bias voltage are a constant. This is true for a standard collapse mode operated CMUT drum as well as an annular ring CMUT operated in collapse mode.

Additionally, the shape of the channels may be altered to improve the image quality. For example, it is known that using a spiral shape can reduce the number of artifacts in the resulting data, thereby providing improved image quality. More generally, the shape of the channels may be curved, the diameter of the CMUTs may be variable and/or the length of the channels may be variable.

See, for example, Li, X., Gachagan, A., & Murray, P. (2020). Design of 2D sparse array transducers for anomaly detection in medical phantoms. Sensors 2020, 20(18), [5370]. https://doi.org/10.3390/s20185370 where various array configurations/shapes are discussed.

Each channel preferably has more than 3 CMUT drums as this provides a higher signal strength per channel for both transmit and receive.

The manufacturability of the large outer rings, having a diameter up to 30mm could result in loss of yield. A manufacturing method is therefore proposed to remedy the situation, where ring array portions are preferentially etched out of the wafer (DRIE) or separated by means of for example a dicing saw or stealth dicing. Tested and selected ring array portions can thus be assembled by means of a pick and place technique onto a substrate.

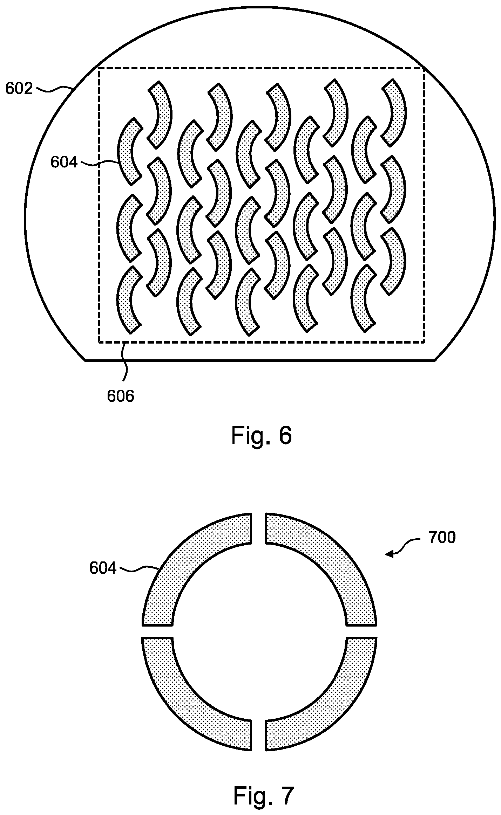

Fig. 6 shows a silicon wafer 602 with ring elements 604. Ring array portions 604 are fabricated in a dense pack manner on the silicon wafer 602. The area in between the segments may be filled by 'dummy CMUT structures' to improve the processing uniformity. For example, the area 604 may be filled with either dummy CMUT structures or with the CMUT drums in the areas comprising the ring array portions 604. Dummy CMUT structures may not have connections to other CMUT structures or may not have electrodes.

For example, DRIE etching is used to separate the segments to enable curved ring array portions 604 to be obtained. DRIE etching can be combined with back side grinding to separate the ring array portions on the silicon wafer. After testing, the ring array portions are combined on a substrate by a 'pick and place' system, to assemble the ring array.

Fig. 7 shows an assembled ring array 700 with four ring array portions 604. The substrate is not shown. More than four ring arrays may be preferred in order to improve the packing of the ring array portions 604 on the silicon wafer.

Drawbacks of manufacturing ring arrays typically include low yields and high usage of silicon.

The proposed assembly overcomes the typical drawbacks of manufacturing ring array because the ring array elements 604 are individually picked and placed in the ring pattern. This increases the yield whilst reducing the use of silicon.

The arrangement may, for example, use square wafer fragments. A complete circular window can be placed over the ring array portions 604 in order to form a homogeneous face to the part to be imaged.

The substrate (e.g., a PCB) may be made of a non-flexible or a flexible substrate, depending on the end application requirements.

It will be appreciated that this manufacturing method can be used for both single ring arrays and multi-ring arrays (e.g., double ring arrays).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

The term CMUT used in this application stands for capacitive micromachined ultrasonic transducer.

If the term "adapted to" is used in the claims or description, it is noted the term "adapted to" is intended to be equivalent to the term "configured to".

Any reference signs in the claims should not be construed as limiting the scope.

## Claims

1. A transducer ring array for volumetric ultrasound imaging, the ring array comprising:
a set of radially extending first channels (301), wherein each first channel (301) comprises a plurality of outer CMUT drums (306), a plurality of inner CMUT drums (308), and a respective radiofrequency, RF, line (304) that connects to the pluralities of outer and inner CMUT drums (306, 308);
a set of radially extending second channels (302), wherein each second channel (302) comprises only a plurality of outer CMUT drums (306), and a respective radiofrequency, RF, line (304) that connects to the plurality of outer CMUT drums (306); and
a biasing system comprising, for each of the first channels (301), a biasing line (310) for applying a bias to the plurality of outer CMUT drums (306), and a biasing line (310) for biasing the plurality of inner CMUT drums (308) that is individually addressable from the biasing line (310) for applying a bias to the plurality of outer CMUT drums (306), or wherein the biasing system comprises a circuit configured to be capable of only providing a bias to either the plurality of outer CMUT drums (306) or the plurality of inner CMUT drums (308);
the biasing system (310) further comprising, for each of the second channels (302), a biasing line (310) connected to the plurality of outer CMUT drums (306) for applying a bias to the plurality of outer CMUT drums (306);
**characterized in that**
each second channel (302) is arranged in between a pair of first channels (301);
wherein the plurality of outer CMUT drums (306) of both the first channels (301) and the second channels (302) form an outer ring (312), and wherein the plurality of inner CMUT drums (308) of the first channels (301) form an inner ring (314), wherein an inner diameter of the outer ring (312) is larger than an outer diameter of the inner ring (314).

2. The transducer ring array of claim 1, wherein an outer CMUT drum (306) among the plurality of outer CMUT drums (306) that is positioned farther from the plurality of inner CMUT drums (308) has a larger diameter than an outer CMUT drum (306) among the plurality of outer CMUT drums (306) that is positioned closer to the plurality of inner CMUT drums (308).

3. The transducer ring array of claim 2, wherein an inner CMUT drum (308) among the plurality of inner CMUT drums (308) that is positioned closer to the plurality of outer CMUT drums (306) has a larger diameter than an inner CMUT drum (308) among the plurality of inner CMUT drums (308) that is postioned farther from the plurality of outer CMUT drums (306).

4. The transducer ring array of any of claims 1 to 3, wherein the first channels (301) comprise three or more outer CMUT drums (306) and three or more inner CMUT drums (308) and wherein the second channels (302) comprise three of more outer CMUT drums (306).

5. An ultrasound imaging system comprising:
the transducer ring array of any of claims 1 to 4; and
a controller configured to:
obtain nearby ultrasound data from the plurality of inner CMUT drums (308) of the first channels (301) by applying a bias to the plurality of inner CMUT drums (308);
obtain faraway ultrasound data from the plurality of outer CMUT drums (306) of the first channels (301) and the plurality of outer CMUT drums (306) of the second channels (302) by applying a bias to the plurality of outer CMUT drums (306) of the first channels (301) and second channels (302); and
generate a compound, three dimensional, ultrasound image by combining the nearby ultrasound data and the faraway ultrasound data.

6. A method of manufacturing a transducer ring array, the method **characterized by** comprising:
generating ring array portions (604) on a silicon wafer (602), the ring array portions comprising a plurality of radially extending first channels (301), each first channel (301) comprising a plurality of outer CMUT drums (306) and a plurality of inner CMUT drums (308), and a plurality of radially extending second channels (302), each second channel (302) comprising only a plurality of outer CMUT drums (306); and
combining two or more ring array portions to generate the transducer ring array;
wherein each second channel (302) is arranged in between a pair of first channels (301).

7. The method of claim 6, further comprising combining four or more ring array portions (604) to generate the transducer ring array.

8. The method of claims 6 or 7, further comprising separating the ring array portions (604) from the silicon wafer (602) by using deep reactive ion etching, DRIE, to etch the silicon wafers (602) and grinding the etched silicon wafers (602) to release the ring array portions (604).

9. The method of any of claims 6 to 8 further comprising testing the plurality of first channels (301) and the plurality of second channels (302) to identify acceptable ring array portions (604) and combining two or more of the acceptable ring array portions (604) to generate the transducer ring array.

10. The method of any of claims 6 to 9, wherein combining the two or more ring array portions (604) comprises placing the ring array portions (604) in a ring pattern.

11. The method of any of claims 6 to 10, wherein combining the two or more ring array portions (604) comprises placing the ring array portions (604) on a substrate, the substrate comprising:
for each first channel (301), an RF line (304) connecting the plurality of outer CMUT drums (306) and the plurality of inner CMUT drums (308), and for each second channel (302), an RF line (304) connecting the plurality of outer CMUT drums (306);
a biasing system comprising, for each of the first channels (301), a biasing line (310) for applying a bias to the plurality of outer CMUT drums (306), and a biasing line (310) for biasing the plurality of inner CMUT drums (308) that is individually addressable from the biasing line (310) for applying a bias to the plurality of outer CMUT drums (306), or wherein the biasing system comprises a circuit configured to be capable of only providing a bias to either the plurality of outer CMUT drums (306) or the plurality of inner CMUT drums (308);
the biasing system (310) further comprising, for each of the second channels (302), a biasing line (310) connected to the plurality of outer CMUT drums (306) for applying a bias to the plurality of outer CMUT drums (306).

## Patentansprüche

1. Wandler-Ring-Array für eine volumetrische Ultraschallbildgebung, das Ring-Array umfassend:
einen Satz von sich radial erstreckenden ersten Kanälen (301), wobei jeder erste Kanal (301) eine Vielzahl von äußeren CMUT-Trommeln (306), eine Vielzahl von inneren CMUT-Trommeln (308) und eine jeweilige Hochfrequenzleitung, HF-Leitung, (304) umfasst, die mit der Vielzahl der äußeren und der inneren CMUT-Trommeln (306, 308) verbunden ist;
einen Satz von sich radial erstreckenden zweiten Kanälen (302), wobei jeder zweite Kanal (302) nur eine Vielzahl von äußeren CMUT-Trommeln (306) und eine jeweilige Hochfrequenzleitung, HF-Leitung, (304) umfasst, die mit der Vielzahl von äußeren CMUT-Trommeln (306) verbunden ist; und
ein Vorspannsystem, umfassend für jeden der ersten Kanäle (301) eine Vorspannleitung (310) zum Anlegen einer Vorspannung an die Vielzahl von äußeren CMUT-Trommeln (306) und eine Vorspannleitung (310) zum Vorspannen der Vielzahl von inneren CMUT-Trommeln (308), die von der Vorspannleitung (310) zum Anlegen einer Vorspannung an die Vielzahl von äußeren CMUT-Trommeln (306) individuell adressierbar ist, oder wobei das Vorspannsystem eine Schaltung umfasst, die konfiguriert ist, um imstande zu sein, eine Vorspannung nur an entweder die Vielzahl von äußeren CMUT-Trommeln (306) oder die Vielzahl von inneren CMUT-Trommeln (308) bereitzustellen;
das Vorspannsystem (310) ferner umfassend für jeden der zweiten Kanäle (302) eine mit der Vielzahl von äußeren CMUT-Trommeln (306) verbundene Vorspannleitung (310) zum Anlegen einer Vorspannung an die Vielzahl von äußeren CMUT-Trommeln (306);
**dadurch gekennzeichnet, dass** jeder zweite Kanal (302) zwischen einem Paar erster Kanäle (301) angeordnet ist;
wobei die Vielzahl von äußeren CMUT-Trommeln (306) sowohl der ersten Kanäle (301) als auch der zweiten Kanäle (302) einen äußeren Ring (312) ausbildet und wobei die Vielzahl von inneren CMUT-Trommeln (308) der ersten Kanäle (301) einen inneren Ring (314) ausbildet, wobei ein innerer Durchmesser des äußeren Rings (312) größer als ein äußerer Durchmesser des inneren Rings (314) ist.

2. Wandler-Ring-Array nach Anspruch 1, wobei eine äußere CMUT-Trommel (306) unter der Vielzahl von äußeren CMUT-Trommeln (306), die weiter entfernt von der Vielzahl von inneren CMUT-Trommeln (308) positioniert ist, einen größeren Durchmesser als eine äußere CMUT-Trommel (306) unter der Vielzahl von äußeren CMUT-Trommeln (306), die näher an der Vielzahl von inneren CMUT-Trommeln (308) positioniert ist, aufweist.

3. Wandler-Ring-Array nach Anspruch 2, wobei eine innere CMUT-Trommel (308) unter der Vielzahl von inneren CMUT-Trommeln (308), die näher an der Vielzahl von äußeren CMUT-Trommeln (306) positioniert ist, einen größeren Durchmesser als eine innere CMUT-Trommel (308) unter der Vielzahl von inneren CMUT-Trommeln (308), die weiter entfernt von der Vielzahl von äußeren CMUT-Trommeln (306) positioniert ist, aufweist.

4. Wandler-Ring-Array nach einem der Ansprüche 1 bis 3, wobei die ersten Kanäle (301) drei oder mehr äußere CMUT-Trommeln (306) und drei oder mehr innere CMUT-Trommeln (308) umfassen und wobei die zweiten Kanäle (302) drei oder mehr äußere CMUT-Trommeln (306) umfassen.

5. Ultraschallbildgebungssystem, umfassend:
das Wandler-Ring-Array nach einem der Ansprüche 1 bis 4; und
eine Steuervorrichtung, die konfiguriert ist zum:
Erhalten nahegelegener Ultraschalldaten von der Vielzahl von inneren CMUT-Trommeln (308) der ersten Kanäle (301) durch Anlegen einer Vorspannung an die Vielzahl von inneren CMUT-Trommeln (308);
Erhalten ferngelegener Ultraschalldaten von der Vielzahl von äußeren CMUT-Trommeln (306) der ersten Kanäle (301) und der Vielzahl von äußeren CMUT-Trommeln (306) der zweiten Kanäle (302) durch Anlegen einer Vorspannung an die Vielzahl von äußeren CMUT-Trommeln (306) der ersten Kanäle (301) und der zweiten Kanäle (302); und
Erzeugen eines zusammengesetzten, dreidimensionalen Ultraschallbilds durch Kombinieren der nahegelegenen Ultraschalldaten und der ferngelegenen Ultraschalldaten.

6. Verfahren zum Herstellen eines Wandler-Ring-Arrays, wobei das Verfahren **dadurch gekennzeichnet ist, dass** es umfasst:
Erzeugen von Ring-Array-Abschnitten (604) auf einem Silizium-Wafer (602), die Ring-Array-Abschnitte umfassend eine Vielzahl von sich radial erstreckenden ersten Kanälen (301), jeder erste Kanal (301) umfassend eine Vielzahl von äußeren CMUT-Trommeln (306) und eine Vielzahl von inneren CMUT-Trommeln (308), und eine Vielzahl von sich radial erstreckenden zweiten Kanälen (302), jeder zweite Kanal (302) umfassend nur eine Vielzahl von äußeren CMUT-Trommeln (306); und
Kombinieren von zwei oder mehr Ring-Array-Abschnitten, um das Wandler-Ring-Array zu erzeugen;
wobei jeder zweite Kanal (302) zwischen einem Paar erster Kanäle (301) angeordnet ist.

7. Verfahren nach Anspruch 6, ferner umfassend das Kombinieren von vier oder mehr Ring-Array-Abschnitten (604), um das Wandler-Ring-Array zu erzeugen.

8. Verfahren nach Anspruch 6 oder 7, ferner umfassend ein Trennen der Ring-Array-Abschnitte (604) von dem Silizium-Wafer (602) durch Verwenden von reaktivem Ionentiefenätzen, DRIE, um die Silizium-Wafer (602) zu ätzen, und Schleifen der geätzten Silizium-Wafer (602), um die Ring-Array-Abschnitte (604) freizugeben.

9. Verfahren nach einem der Ansprüche 6 bis 8, ferner umfassend ein Testen der Vielzahl von ersten Kanälen (301) und der Vielzahl von zweiten Kanälen (302), um akzeptable Ring-Array-Abschnitte (604) zu identifizieren, und das Kombinieren von zwei oder mehr der akzeptablen Ring-Array-Abschnitte (604), um das Wandler-Ring-Array zu erzeugen.

10. Verfahren nach einem der Ansprüche 6 bis 9, wobei das Kombinieren der zwei oder mehr Ring-Array-Abschnitte (604) ein Platzieren der Ring-Array-Abschnitte (604) in einem Ringmuster umfasst.

11. Verfahren nach einem der Ansprüche 6 bis 10, wobei das Kombinieren der zwei oder mehr Ring-Array-Abschnitte (604) das Platzieren der Ring-Array-Abschnitte (604) auf einem Substrat umfasst, das Substrat umfassend:
für jeden ersten Kanal (301) eine HF-Leitung (304), die die Vielzahl von äußeren CMUT-Trommeln (306) und die Vielzahl von inneren CMUT-Trommeln (308) verbindet, und für jeden zweiten Kanal (302) eine HF-Leitung (304), die die Vielzahl von äußeren CMUT-Trommeln (306) verbindet;
ein Vorspannsystem, umfassend für jeden der ersten Kanäle (301) eine Vorspannleitung (310) zum Anlegen einer Vorspannung an die Vielzahl von äußeren CMUT-Trommeln (306) und eine Vorspannleitung (310) zum Vorspannen der Vielzahl von inneren CMUT-Trommeln (308), die von der Vorspannleitung (310) zum Anlegen einer Vorspannung an die Vielzahl von äußeren CMUT-Trommeln (306) individuell adressierbar ist, oder wobei das Vorspannsystem eine Schaltung umfasst, die konfiguriert ist, um imstande zu sein, eine Vorspannung nur an entweder die Vielzahl von äußeren CMUT-Trommeln (306) oder die Vielzahl von inneren CMUT-Trommeln (308) bereitzustellen;
das Vorspannsystem (310) ferner umfassend für jeden der zweiten Kanäle (302) eine mit der Vielzahl von äußeren CMUT-Trommeln (306) verbundene Vorspannleitung (310) zum Anlegen einer Vorspannung an die Vielzahl von äußeren CMUT-Trommeln (306).

## Revendications

1. Réseau d'anneaux de transducteur pour une imagerie volumétrique par ultrasons, le réseau d'anneaux comprenant :
un ensemble de premiers canaux s'étendant radialement (301), dans lequel chaque premier canal (301) comprend une pluralité de tambours CMUT externes (306), une pluralité de tambours CMUT internes (308), et une ligne de radiofréquence, RF (304) respective qui se connecte aux pluralités de tambours CMUT externes et internes (306, 308) ;
un ensemble de seconds canaux s'étendant radialement (302), dans lequel chaque second canal (302) ne comprend qu'une pluralité de tambours CMUT externes (306), et une ligne de radiofréquence, RF (304) respective qui se connecte à la pluralité de tambours CMUT externes (306) ; et
un système de sollicitation comprenant, pour chacun des premiers canaux (301), une ligne de sollicitation (310) permettant d'appliquer une sollicitation à la pluralité de tambours CMUT externes (306), et une ligne de sollicitation (310) permettant de solliciter la pluralité de tambours CMUT internes (308) qui est adressable individuellement à partir de la ligne de sollicitation (310) permettant d'appliquer une sollicitation à la pluralité de tambours CMUT externes (306), ou dans lequel le système de sollicitation comprend un circuit configuré pour être capable de ne fournir une sollicitation qu'à la pluralité de tambours CMUT externes (306) ou à la pluralité de tambours CMUT internes (308) ;
le système de sollicitation (310) comprenant en outre, pour chacun des seconds canaux (302), une ligne de sollicitation (310) reliée à la pluralité de tambours CMUT externes (306) permettant d'appliquer une sollicitation à la pluralité de tambours CMUT externes (306) ;
**caractérisé en ce que** chaque second canal (302) est agencé entre une paire de premiers canaux (301) ;
dans lequel la pluralité de tambours CMUT externes (306) à la fois des premiers canaux (301) et des seconds canaux (302) forment un anneau externe (312), et dans lequel la pluralité de tambours CMUT internes (308) des premiers canaux (301) forment un anneau interne (314), dans lequel un diamètre interne de l'anneau externe (312) est plus grand qu'un diamètre externe de l'anneau interne (314).

2. Réseau d'anneaux de transducteur selon la revendication 1, dans lequel un tambour CMUT externe (306) parmi la pluralité de tambours CMUT externes (306) qui est positionné plus loin de la pluralité de tambours CMUT internes (308) a un diamètre plus grand qu'un tambour CMUT externe (306) parmi la pluralité de tambours CMUT externes (306) qui est positionné plus près de la pluralité de tambours CMUT internes (308).

3. Réseau d'anneaux de transducteur selon la revendication 2, dans lequel un tambour CMUT interne (308) parmi la pluralité de tambours CMUT internes (308) qui est positionné plus près de la pluralité de tambours CMUT externes (306) a un diamètre plus grand qu'un tambour CMUT interne (308) parmi la pluralité de tambours CMUT internes (308) qui est positionné plus loin de la pluralité de tambours CMUT externes (306).

4. Réseau d'anneaux de transducteur selon l'une quelconque des revendications 1 à 3, dans lequel les premiers canaux (301) comprennent trois tambours CMUT externes (306) ou plus et trois tambours CMUT internes (308) ou plus, et dans lequel les seconds canaux (302) comprennent trois tambours CMUT externes (306) ou plus.

5. Système d'imagerie par ultrasons comprenant :
le réseau d'anneaux de transducteur selon l'une quelconque des revendications 1 à 4 ; et
un dispositif de commande configuré pour :
obtenir des données ultrasonores à proximité à partir de la pluralité de tambours CMUT internes (308) des premiers canaux (301) en appliquant une sollicitation à la pluralité de tambours CMUT internes (308) ;
obtenir des données ultrasonores éloignées à partir de la pluralité de tambours CMUT externes (306) des premiers canaux (301) et de la pluralité de tambours CMUT externes (306) des seconds canaux (302) en appliquant une sollicitation à la pluralité de tambours CMUT externes (306) des premiers canaux (301) et des seconds canaux (302) ; et
générer une image ultrasonore composée tridimensionnelle en combinant les données ultrasonores proches et les données ultrasonores éloignées.

6. Procédé de fabrication d'un réseau d'anneaux de transducteur, le procédé **étant caractérisé en ce qu'il** comprend :
la génération de parties de réseau annulaire (604) sur une tranche de silicium (602), les parties de réseau annulaire comprenant une pluralité de premiers canaux s'étendant radialement (301), chaque premier canal (301) comprenant une pluralité de tambours CMUT externes (306) et une pluralité de tambours CMUT internes (308), et une pluralité de seconds canaux s'étendant radialement (302), chaque second canal (302) comprenant uniquement une pluralité de tambours CMUT externes (306) ; et
la combinaison de deux parties de réseau annulaire ou plus pour générer le réseau annulaire de transducteur ;
dans lequel chaque second canal (302) est agencé entre une paire de premiers canaux (301).

7. Procédé selon la revendication 6, comprenant en outre la combinaison de quatre parties de réseau annulaire ou plus (604) pour générer le réseau annulaire de transducteur.

8. Procédé selon les revendications 6 ou 7, comprenant en outre la séparation des parties de réseau annulaire (604) de la tranche de silicium (602) en utilisant une gravure par ions réactifs profonde, DRIE, pour graver les tranches de silicium (602) et broyer les tranches de silicium gravées (602) pour libérer les parties de réseau annulaire (604).

9. Procédé selon l'une quelconque des revendications 6 à 8, comprenant en outre le test de la pluralité de premiers canaux (301) et la pluralité de seconds canaux (302) pour identifier des parties de réseau annulaire (604) acceptables, et la combinaison de deux ou plusieurs des parties de réseau annulaire acceptables (604) pour générer le réseau annulaire de transducteur.

10. Procédé selon l'une quelconque des revendications 6 à 9, dans lequel la combinaison des deux parties de réseau annulaire (604) ou plus comprend le placement des parties de réseau annulaire (604) dans un modèle d'anneau.

11. Procédé selon l'une quelconque des revendications 6 à 10, dans lequel la combinaison des deux parties de réseau annulaire (604) ou plus comprend le placement des parties de réseau annulaire (604) sur un substrat, le substrat comprenant :
pour chaque premier canal (301), une ligne RF (304) connectant la pluralité de tambours CMUT externes (306) et la pluralité de tambours CMUT internes (308), et pour chaque second canal (302), une ligne RF (304) reliant la pluralité de tambours CMUT externes (306) ;
un système de sollicitation comprenant, pour chacun des premiers canaux (301), une ligne de sollicitation (310) permettant d'appliquer une sollicitation à la pluralité de tambours CMUT externes (306), et une ligne de sollicitation (310) permettant de solliciter la pluralité de tambours CMUT internes (308) qui est adressable individuellement à partir de la ligne de sollicitation (310) permettant d'appliquer une sollicitation à la pluralité de tambours CMUT externes (306), ou dans lequel le système de sollicitation comprend un circuit configuré pour être capable de ne fournir une sollicitation qu'à la pluralité de tambours CMUT externes (306) ou à la pluralité de tambours CMUT internes (308) ;
le système de sollicitation (310) comprenant en outre, pour chacun des seconds canaux (302), une ligne de sollicitation (310) reliée à la pluralité de tambours CMUT externes (306) permettant d'appliquer une sollicitation à la pluralité de tambours CMUT externes (306).
